Europäisches Patentamt

European Patent Office

Office européen des brevets

(19)

(11) Publication number: **0 180 164**
A2

(12) # EUROPEAN PATENT APPLICATION

(21) Application number: **85113631.7**

(22) Date of filing: **28.10.85**

(51) Int. Cl.⁴: **C 12 N 9/36**

(30) Priority: **26.10.84 HU 398384**

(71) Applicant: **REANAL FINOMVEGYSZERGYAR, P.O. Box 54 Telepes u. 53, H-1441 Budapest (HU)**

(72) Inventor: **Bánky, Bulcsu, Bakator u. 3, H-1118 Budapest (HU)**
Inventor: **Csóka, Arpád, Szentendrei u. 14, H-1035 Budapest (HU)**
Inventor: **Csomós, Andor, Kolozsvár u. 36/b, H-1151 Budapest (HU)**
Inventor: **Jécsai, György, Révész u. 2/c, H-1138 Budapest (HU)**
Inventor: **László, Elemér, Ihász köz, 8, H-1105 Budapest (HU)**
Inventor: **Pálmai, György, Biró u. 17/b, H-1122 Budapest (HU)**

(43) Date of publication of application: **07.05.86 Bulletin 86/19**

(84) Designated Contracting States: **AT BE CH DE FR GB IT LI LU NL SE**

(74) Representative: **Beszédes, Stephan G., Dr., Am Heideweg 2 Postfach 1168, D-8060 Dachau (DE)**

(54) **Method for the preparation of lysozyme enzyme.**

(57) The application is concerned with a method for the preparation of lysozyme enzyme and its salts form an egg-white solution with a pH value of 6 to 8 by binding lysozyme on a weak acidic cation exchanger, separating the cation exchanger from the protein solution, washing the cation exchanger, eluting lysozyme from the cation exchanger and crystallizing lysozyme from the eluate as free base or as its acid addition salt and optionally regenerating the cation exchanger, in which as a weak acidic cation exchanger a weak acidic cation exchanger gel amidated on its surface and smaller in pore diameter than an average exclusion molecular weight of 25,000, there is used for the washing of the gel an aqueous alkali halide solution containing up to 1 w/v % of an alkali halide, and the elution of the enzyme is carried out with a 4 to 15 w/v % aqueous alkali halide solution.

The invention is concerned with a new method for the preparation of lysozyme enzyme and its salts from egg-white.

It is known that lysozyme enzyme, present in egg-white, is a strongly basic protein with an isoelectric point of pH value of 10.5 to 11. The molecular weight of the enzyme is 14,300. Lysozyme is a white, crystalline substance, well soluble in water and dilute buffers, but practically insoluble in organic liquids. The aqueous solution of the enzyme is relatively thermally stable, and it can be stored for an arbitrary period at 2 to 4°C under aseptic conditions.

Owing to its antibacterial effects, lysozyme is utilized in the pharmaceutical industry primarily as an active agent of anti-inflammatory preparations (such as ointments) and in the food industry as a preservative. In these fields of application a highly pure enzyme, with a specific activity of at least 25,000 Shugar units/mg, is required (Viskhim. Biophys. Acta 8 [1952], 303). However, the crystalline enzyme in the required purity can be prepared only by very sophisticated and expensive methods which impedes the widespread use of lysozyme.

Several methods have been disclosed for the separation of lysozyme from egg-white. In all of these known methods egg-white is separated from the yolk, the viscosity of the resulting solution is lowered by adjusting its pH value, and then the solid particles (such as chalaza) are removed from the solution. Thereafter, according to one of the known methods (J. Biol. Chem. 157 [1945], 43), the pH value of the solution is adjusted to about the isoelectric point by adding an alkali, and the enzyme is salted out with sodium chloride. The enzyme is then purified by crystallization at room temperature. This method has the disadvantages that crystallization is lengthy, requiring sometimes even one week, the purity grade of the resulting product is insufficient, and the protein lye obtained as by-product and containing at least 5% by weight of sodium chloride is useless.

The methods disclosed in DOS 2,248,843 and 2,248,804, applying multiple ultrafiltration steps, are lengthy and require expensive equipment. It is a further disadvantage that the filtrate does not contain the other protein components of egg-white. Again, lysozyme is separated from the concentrate by salting out, utilizing a 40% by weight of aqueous ammonium sulfate solution.

According to the method described in British patent No. 1,110,466 the pH value of egg-white solution is adjusted to 6.5, thereafter about 30% by weight of a weak acidic ion exchange resin (Amberlite® IRC-50) is added, and the mixture is stirred at 0 to 5°C. After the bulk of lysozyme has been bound to the resin, the resin is separated from the protein solution, lysozyme is eluted with a 8% aqueous ammonium sulfate solution , and further amounts of ammonium sulfate are added to the eluate to precipitate the enzyme. In contrast to the other known methods, this procedure enables one to

- 4 -

utilize the protein components of egg-white which remain back after the separation of the enzyme. This method has, however, the following disadvantages:

a) The weak acidic ion exchange resin binds, in addition to lysozyme, the other alkaline components of egg-white, too, which are eluted together with lysozyme, lowering thereby the purity grade of the enzyme separated;

b) the method requires relatively high amounts of ion exchange resin;

c) in the elution step a dilute enzyme solution is obtained, from which lysozyme can be isolated with a relatively low efficiency;

d) the protein solution is treated with the resin at 0 to 5°C, which requires much energy for cooling.

The problem underlying to the invention is to elaborate of a new method for the preparation of lysozyme enzyme, free of the disadvantages of the known ones, which enables one to separate highly pure lysozyme from egg-white in a simple and efficient way, without significantly decreasing the amount of the other valuable components of egg-white, and to provide a protein solution which can be further processed to yield valuable protein products.

Surprisingly the above has been attained by the invention. The invention is based on the recognition

- 5 -

that weak acidic cation exchanger gels amidated on their surface and smaller in pore diameter than an average exclusion molecular weight of 25,000 bind lysozyme selectively from egg-white solutions. This recognition is very surprising since no substance which is able to bind practically only one protein from a multicomponent protein solution has been known before.

The invention is also based on the recognition that lysozyme can be eluted from the separated and washed gel with a relatively low amount of a 4 to 15 w/v % aqueous alkali halide solution. In this step a concentrated enzyme solution is obtained, from which lysozyme can be crystallized very easily at room temperature.

Hence the subject-matter of the invention is a method for the preparation of lysozyme enzyme and its salts from an egg-white solution with a pH value of 6 to 8 by binding lysozyme on a weak acidic cation exchanger, separating the cation exchanger from the protein solution, washing the cation exchanger, eluting lysozyme from the cation exchanger and crystallizing lysozyme from the eluate as free base or as its acid addition salt and optionally regenerating the cation exchanger, which is characterized in that there is used as a weak acidic cation exchanger a weak acidic cation exchanger gel amidated on its surface and smaller in pore diameter than an average exclusion molecular weight of 25,000, there is used for the washing of the gel an aqueous alkali halide solution containing up to 1 w/v % of an alkali halide and the elution of the enzyme is carried out with a 4 to 15 w/v % aqueous alkali halide solution.

Preferably a carboxymethyl dextran gel or a carboxymethyl cellulose gel (i.e. dextran gel or cellulose gel modified with carboxymethyl groups) amidated on its surface (i.e. in which the surface carboxy groups are present in amidated form), is utilized as a cation exchanger gel. Surface amidation could have been performed by dispersing the swollen gel particles in an organic liquid, and reacting them with dicyclohexyl carbodiimide and ethanol amine. Of the gel types mentioned above, for example CM-25 type ion exchanger gels possess the required pore diameter, smaller than an average exclusion molecular weight of 25,000. Although surface amidation lowers the ion exchange capacity of the gel by about 10 to 20 %, it ensures that basic proteins with higher molecular weights than the exclusion value do not bind to the gel surface by ionic bonds.

Furthermore it is preferred that 1 to 5 % by weight, based on the weight of the egg-white solution, of cation exchanger gel are added to the egg-white solution and the resulting suspension is stirred before separating it from the egg-white solution. Preferably stirring of the suspension is carried for at least 1 hour. Moreover preferably stirring of the suspension is carried out at room temperature.

According to a preferred embodiment of the invention one proceeds as follows:

The protein solution is passed through a column filled with a gel specified above, this, however, requires a relatively long time. Hence according to a more preferred embodiment the cation exchange gel is added to the protein solution, and the suspension is stirred for at least one hour, generally for about 1 to 3 hours. It has been observed that lysozyme binds completely to the gel within this period. Rather low amounts of the wet cation exchanger gel (1 to 5 % by weight, based on the weight of the protein solution) are sufficient to bind the total amount of lysozyme present.

After this binding step the gel column (in this case the protein solution separates from the gel in passing through it) or the gel separated from the protein solution, optionally after drying, is washed with a dilute aqueous alkali halide solution (generally with a 0.2 to 1 w/v % aqueous sodium chloride solution) to remove foreign proteins, and then the gel is eluted with a 4 to 15 w/v %, preferably 5 to 10 w/v %, aqueous alkali halide solution. It is preferred to use aqueous sodium chloride solution in this step. It has been observed that 2 to 5 parts by volume of eluting agent, based on 1 part by volume of the gel, are generally sufficient to remove lysozyme enzyme completely. This amount of the aqueous alkali halide solution is applied onto the gel preferably in multiple portions.

Lysozyme is crystallized from the resulting eluate as free base or as its acid addition salt. Crystallization can be performed in a manner known per se. When lysozyme is to be separated as free base, one proceeds preferably as follows: The pH value of the eluate is adjusted to 9 to 10 by adding a base, then, if necessary, the solution is seeded with crystalline lysozyme, and the mixture is stirred. The separated crystals are filtered off. Lysozyme is obtained in high purity.

When lysozyme is to be separated as its acid addition salt, the salt-forming acid can be added to the eluate, whereafter preferably one proceeds as decribed above.

If desired, the cation exchange gel used in the process of the invention can be regenerated and then used again. Thus according a preferred optional embodiment of the invention the regeneration of the cation exchanger gel is carried out by suspending it in water and stirring the suspension at room temperature for at least 12 hours in the presence of an alkalase enzyme.

In detail according to this embodiment of the invention most preferably one proceeds as follows.

The clustered gel beads are suspended in water, suitably after adjusting the suspension to an alkaline pH value, preferably of 8 to 9, for example, 8.5, an alkalase enzyme is added to the suspension, and the suspension is stirred at room temperature for at least 12 hours. The alkalase enzyme is inactivated with hydrogen peroxide, thereafter the gel beads are filtered off and converted into the acidic ($H^+$) form. The resulting regenerated gel can be utilized repeatedly in the process of the invention.

The major advantages of the process of the invention are as follows:

A) Lysozyme enzyme can be separated selectively in a single operation from the other components of egg-white.

B) Lysozyme crystallized from the eluate is of high purity, its specific activity is at least 25,000 Shugar units/mg. By the known methods such a purity could be attained only after several recrystallizations.

C) The method is simple, quick, efficient, and requires a minimum energy.

D) Very small amounts of ion exchanger gel are sufficient to bind lysozyme completely.

E) The ion exchanger gel once used in the separation of lysozyme can be regenerated very easily and can be used repeatedly in several steps.

F) The method of the invention can be adapted very easily to any complex egg-processing technology, since no foreign substance is added to egg-white, only insignificant amounts of substances other than lysozyme are removed from egg-white, and the amount of egg-white solution decreases only to a minimum extent. The lysozyme-free egg-white solution can be processed further to obtain valuable products.


The process of the invention is elucidated in detail by means of the following Examples.

## Example 1

a) Separation of lysozyme enzyme from egg-white solution

100 kg of egg-white solution are used as starting substance. The pH value of the solution is adjusted to 6.5, and 3 000 ml of the wet, amidated carboxymethyl dextran gel [Molselect® CM-25 gel], prepared as described below, are added to the solution under slow stirring. The suspension is stirred slowly at room temperature for 2 hours, thereafter stirring is stopped, and the gel is allowed to settle for 2 hours. The supernatant, about 92 litres in volume, is decanted and spray-dried. 12,880 g of dry egg-white powder are obtained.

The gel is filled into a large sintered glass filter, suctioned, and then washed with a 0.5 w/v % aqueous sodium chloride solution until the wash is free of proteins. The protein content of the wash is examined with a 20 w/v % aqueous trichloroacetic acid solution; the wash is regarded free of proteins when no precipitation can be observed. About 4 parts by volume of the aqueous sodium chloride solution, calculated on 1 part by volume of the wet gel, are sufficient to wash out the proteins.

Thereafter the washed gel is eluted with a 5 w/v % aqueous sodium chloride solution to remove lysozyme. A total amount of 8,000 ml of sodium chloride solution is used, this amount of the solution is applied onto the gel in 3 to 4 portions. The dissolution of lysozyme is examined with trichloroacetic acid as described above.

The eluate is filtered on a sintered glass filter, the pH value of the filtrate is adjusted to 10.0 with 2 n aqueous sodium hydroxide solution, thereafter the mixture is seeded with crystalline lysozyme, and lysozyme is crystallized for 24 hours. 224 g of crystalline lysozyme base are obtained; specific activity: 26,000 Shugar units/mg.

b) Regeneration of the cation exchanger gel

The used, clustered gel is suspended in about two parts by volume of water, the pH value of the suspension is adjusted to 8.5 with 3 n aqueous sodium hydroxide solution, and then 0.1 w/v % of alkalase enzyme (obtained from Boc. licheniformis, produced by NOVO; specific activity: 1.5-2.0 Anson units/g) and 0.1 v/v % of anionic detergent [Tween 80] are dissolved in the suspension. The suspension is stirred at room temperature for 24 hours, then the alkalase enzyme is inactivated with 0.1 v/v % of a 30% aqueous hydrogen peroxide solution. The mixture is allowed to stand at room temperature for 8 hours, thereafter the gel is filtered off on a sintered glass filter and treated with 1 n aqueous sodium hydroxide solution. The resulting gel, in Na$^+$ form, is washed thoroughly with distilled water, and then treated with 1 n aqueous hydrochloric acid solution to convert it into the H$^+$ form. Finally, the gel is washed chloride-free with distilled water.

This regenerated gel can be stored at room temperature under aseptic conditions as a distilled water suspension.

The amidated carboxymethyl dextran gel [Molselect® CM-25 gel] used as a binding agent has been prepared as follows.

Surface amidation of the carboxymethyl dextran gel

Wet carboxymethyl dextran gel [Molselect® CM-25 gel] in H$^+$ form is used as a starting substance. An amount of the wet gel corresponding to 1 000 g of dry gel is suspended in 7 500 ml of toluene, and 240 g of dicyclohexyl carbodiimide and 100 g of ethanol amine are added to the suspension. The suspension is stirred slowly at room temperature for 20 hours, thereafter the gel is filtered off, washed in some steps with toluene, then suspended in distilled water, and the suspension is allowed to settle. This aqueous treatment is repeated at least twice more. Thereafter the gel is filtered off, washed three times with 0.5 n aqueous hydrochloric acid on the filter to bring it into H$^+$

form, and the excess of hydrochloric acid is washed out with distilled water. The pH value of the wash is periodically tested with an indicator paper, and washing is continued until the wash is neutral. About 3 000 ml of wet, amidated carboxymethyl dextran gel [Molselect(R) CM-25 gel] are obtained.


### Example 2

7 kg of a carboxymethyl dextran cation exchange gel [Sephadex(R) CM-25] amidated as described at the end of Example 1, and then washed thoroughly with distilled water are used as a starting substance. 19.8 litres of the resulting wet gel are added with stirring to 1 000 kg of the egg-white solution (pH value = 6.5), and the suspension is stirred slowly at room temperature for 1 hour. The gel is allowed to settle for 1 hour, then the supernatant, about 920 litres in volume, is decanted and spray-dried. 131.2 kg of egg-white powder are obtained.

The lysozyme-containing gel is washed four times on a filter centrifuge with a total amount of 80 litres of a 0.5 w/v % aqueous sodium chloride solution. Then the resulting protein-free gel is eluted with 95 litres of a 7 w/v % aqueous sodium chloride solution to remove lysozyme. The eluting agent is applied onto the gel in three portions.

The lysozyme-containing solution is filtered, and the filtrate is acidified to a pH value of 4.8 with a 2 n aqueous hydrochloric acid solution. The mixture is maintained at room temperature for 12 hours, and then stirred at +5°C for 12 hours. The separated crystals are removed by centrifuging and dried over phosphorus pentoxide. 3 010 g of lysozyme hydrochloride are obtained; specific activity: 27,800 Shugar units/mg.


## Comparative Example

This Example describes a known method for the separation of lysozyme enzyme from egg-white for comparison purposes.

100 kg of egg-white are stored at +4°C for 3 days, and then the pH is adjusted to 10.0 with 1 n aqueous potassium hydroxide solution. The volume of the solution is measured, and 5 w/v % of solid sodium chloride are dissolved in the solution under vigorous stirring. The solution is seeded with crystalline lysozyme, and lysozyme is crystallized at room temperature for one week. Crystallization can be facilitated by stirring the mixture at room temperature in the daytime, and allowing it to stand at +4°C overnight.

The separated crystals are removed by centrifuging, suspended in 10 litres of cold distilled water, and the pH value of the suspension is adjusted to 5.5 with 1 n aqueous acetic acid solution under stirring. 5 w/v % of solid sodium chloride are dissolved in the resulting solution, then the pH value of the mixture is adjusted to 10.0 with 1 n aqueous potassium hydroxide solution. After 48 hours of standing the resulting lysozyme base is separated and recrystallized as described above. This operation is repeated three times more.

The resulting lysozyme base, recrystallized four times, is dissolved in 5 litres of cold distilled water, the pH value of the solution is adjusted to 5.5 with 1 n aqueous acetic acid solution, the mixture is filtered, and 5 w/v % of solid sodium chloride are dissolved in the filtrate. The solution is cooled to 4°C and acidified to a pH value of 4.5 with aqueous hydrochloric acid. Crystallization is complete within 4 to 5 days. The crystals are filtered off on a sintered glass filter, washed once with ethanol and three times with acetone, and dried at room temperature. 196 g of lysozyme hydrochloride are obtained; specific activity: 22,400 Shugar units/mg.

It clearly results from the above that this comparative product prepared according to a known method notwithstanding to its lengthy and complicated character is inferior to the products of Examples 1 and 2 prepared according to the invention.

Claims

Claims

1.) A method for the preparation of lysozyme enzyme and its salts form an egg-white solution with a pH value of 6 to 8 by binding lysozyme on a weak acidic cation exchanger, separating the cation exchanger from the protein solution, washing the cation exchanger, eluting lysozyme from the cation exchanger and crystallizing lysozyme from the eluate as free base or as its acid addition salt and optionally regenerating the cation exchanger, characterized in that one uses as a weak acidic cation exchanger a weak acidic cation exchanger gel amidated on its surface and smaller in pore diameter than an average exclusion molecular weight of 25,000, one uses for the washing of the gel an aqueous alkali halide solution containing up to 1 w/v % of an alkali halide and one carries out the elution of the enzyme with a 4 to 15 w/v % aqueous alkali halide solution.

2.) A method as claimed in claim 1, characterized in that a carboxymethyl dextran gel or a carboxymethyl cellulose gel, amidated on its surface, is utilized as a cation exchanger gel.

3.) A method as claimed in any of claim 1 or 2, characterized in that 1 to 5 % by weight, based on the weight of the egg-white solution, of cation exchanger gel are added to the egg-white solution and the resulting suspension is stirred before separating it from the egg-white solution.

- 17 -

4.) A method as claimed in any of claims 1 to 3, characterized in that stirring of the suspension is carried for at least 1 hour.

5.) A method as claimed in any of claims 1 to 4, charakterized in that stirring of the suspension is carried out at room temperature.

6.) A method as claimed in claims 1 to 5, characterized in that the regeneration of the cation exchanger gel is carried out by suspending it in water and stirring the suspension at room temperature for at least 12 hours in the presence of an alkalase enzyme.

Abstract